(19) Europäisches Patentamt / European Patent Office / Office européen des brevets

(11) **EP 1 544 674 B1**

(12) **EUROPÄISCHE PATENTSCHRIFT**

(45) Veröffentlichungstag und Bekanntmachung des Hinweises auf die Patenterteilung:
**04.06.2008 Patentblatt 2008/23**

(51) Int Cl.:
**G03B 42/04** *(2006.01)* **G01T 1/29** *(2006.01)*
**G06K 7/00** *(2006.01)*

(21) Anmeldenummer: **03104749.1**

(22) Anmeldetag: **17.12.2003**

(54) **Verfahren zur Bearbeitung eines Bildträgers zur Speicherung von Röntgeninformation**

Method of processing a radiation image storage medium

Méthode de traitement d'un support d'image radiographique

(84) Benannte Vertragsstaaten:
**AT BE BG CH CY CZ DE DK EE ES FI FR GB GR HU IE IT LI LU MC NL PT RO SE SI SK TR**

(43) Veröffentlichungstag der Anmeldung:
**22.06.2005 Patentblatt 2005/25**

(73) Patentinhaber: **Agfa-Gevaert HealthCare GmbH**
**51373 Leverkusen (DE)**

(72) Erfinder:
• **Encke, Walter**
**82205, Gilching (DE)**
• **Haug, Werner**
**81825, München (DE)**
• **Hujer, Oskar**
**85305, Jetzendorf (DE)**

(74) Vertreter: **Linsmeier, Josef**
**Agfa-Gevaert HealthCare GmbH**
**Intellectual Property**
**Tegernseer Landstrasse 161**
**81539 München (DE)**

(56) Entgegenhaltungen:
**EP-A- 0 727 696        EP-A- 1 031 854**
**EP-A- 1 251 683        EP-A- 1 391 780**
**WO-A-02/42794          US-A- 4 320 296**
**US-A- 4 498 005        US-A- 4 739 480**

## Beschreibung

Die Erfindung betrifft ein Verfahren zur Bearbeitung eines Bildträgers zur Aufzeichnung von Röntgenaufnahmen gemäß dem Oberbegriff des Anspruchs 1.

Entsprechende Bildträger werden, insbesondere für medizinische Zwecke, im Bereich der Computer-Radiographie (CR) eingesetzt. Hierbei werden Röntgenaufnahmen in einer Speicherleuchtstoff-Schicht aufgezeichnet, indem die durch ein Objekt, beispielsweise einen Patienten, hindurchtretende Röntgenstrahlung als latentes Bild in der Speicherleuchtstoff-Schicht gespeichert wird. Zum Auslesen der gespeicherten Röntgeninformation wird die Speicherleuchtstoff-Schicht mit Stimulationslicht bestrahlt, wodurch diese zur Aussendung von Emissionslicht angeregt wird, welches von einem optischen Detektor erfasst und in elektrische Signale umgewandelt wird. Die elektrischen Signale können nach Bedarf weiterverarbeitet und auf einem Monitor dargestellt oder an einem entsprechenden Ausgabegerät, wie z. B. einem Drucker, ausgegeben werden. Nach einem Löschvorgang, bei dem etwaige verbliebene Röntgeninformation vollständig aus der Speicherleuchtstoff-Schicht eliminiert wird, steht die Speicherleuchtstoff-Schicht für weitere Röntgenaufnahmen zur Verfügung.

Da Speicherleuchtstoff-Schichten im Allgemeinen unterschiedliche Empfindlichkeiten für Röntgenstrahlung aufweisen, muss bei der Verarbeitung der beim Auslesen des Bildträgers erhaltenen Bildsignale unter anderem die Empfindlichkeit der jeweiligen Speicherleuchtstoff-Schicht für Röntgenstrahlung berücksichtigt werden. Die entsprechenden Daten, die ein Maß für die Empfindlichkeit der Speicherleuchtstoff-Schicht eines Bildträgers für Röntgenstrahlung darstellen, werden im Stand der Technik meist ab Werk auf einem separaten Datenträger mit dem Bildträger mitgeliefert. Dabei kann es bei Verwechslung der einzelnen, im Allgemeinen zu unterschiedlichen Bildträgern gehörenden Datenträger dazu kommen, dass die von einer Speicherleuchtstoff-Schicht eines Bildträgers erhaltenen Bildsignale fälschlicherweise mit den entsprechenden Daten eines anderen Bildträgers korrigiert werden. Dies führt zu einer nicht tolerierbaren Verfälschung der ausgelesenen Röntgeninformation.

EP 1 391 780 A1 offenbart einen Bildträger mit einem Speicherelement, in dem Informationen zur Korrektur der Empfindlichkeit der Speicherleuchtstoffplatte gespeichert werden. In US 4,320,296 wird die Speicherung von Bildverarbeitungsdaten als Maß für die in der Leuchtstoffplatte gespeicherten Röntgendosis beschrieben. Aus EP 1 031 854 A1 ist ein Verfahren zur Bilddatenkorrektur bekannt. EP 1 251 683 A1 offenbart ein Netzwerk zur Übertragung von Bilddaten, die durch Auslesen von Speicherleuchtstoffplatten erhalten wurden. Aus EP 0 727 696 A1 ist die Verwendung von per Radiofrequenzstrahlung auslesbarer Speicherelemente, sog. RF-Tags, bei Röntgenkassetten bekannt. WO 02/42794 A2 offenbart einen integrierten Strahlungsdetektor mit einer Referenz auf einen Kalibrierdatensatz, anhand dessen Empfindlichkeitsschwankungen des Detektors korrigiert werden können.

Bei den aus dem Stand der Technik bekannten Verfahren bzw. Systemen wird aufgrund der großen Menge an Korrekturdaten relativ viel Zeit benötigt, bis diese für die Bilddatenkorrektur bereitstehen. Darüber hinaus kann es zu Übertragungsfehlern kommen.

Es ist Aufgabe der vorliegenden Erfindung, ein Verfahren zur Bearbeitung des Bildträgers anzugeben, bei welchem unterschiedliche Empfindlichkeiten des Bildträgers für Röntgenstrahlung zeitsparend und mit erhöhter Zuverlässigkeit zur Verfügung gestellt werden.

Diese Aufgabe wird durch das Verfahren gemäß Anspruch 1 gelöst.

Das erfindungsgemäße Verfahren zur Bearbeitung des Bildträgers ist dadurch gekennzeichnet, dass der Bildträger einen elektronischen Speicher zur Speicherung von Kalibrierdaten des Bildträgers umfasst, welche ein Maß für die Empfindlichkeit des Bildträgers für Röntgenstrahlung darstellen und bei einer Verarbeitung von Bildsignalen, welche bei einem Auslesen der Röntgeninformation aus dem Bildträger erhalten werden, herangezogen werden können, und die Kalibrierdaten nach einem ersten Auslesen der Kalibrierdaten aus dem elektronischen Speicher des Bildträgers in einem Speicher, insbesondere in einem Zentralspeicher oder in einem Speicher einer Auslesevorrichtung, gespeichert werden.

Bei einer erneuten Bearbeitung desselben Bildträgers werden dann die nach dem ersten Auslesen gespeicherten Kalibrierdaten dieses Bildträgers aus dem Speicher, insbesondere dem Speicher der Auslesevorrichtung bzw. dem Zentralspeicher, gelesen. Die Kalibrierdaten stehen dadurch in kürzerer Zeit für die Bildsignalverarbeitung zur Verfügung als bei einem erneuten Auslesen des zweidimensionalen Datensatzes aus dem elektronischen Speicher des Bildträgers. Auch etwaige Übertragungsfehler beim Auslesen des Datensatzes aus dem elektronischen Speicher können dadurch vermieden werden.

Vorzugsweise erfolgt dabei das erste Auslesen der Kalibrierdaten des Bildträgers aus dem elektronischen Speicher des Bildträgers in der Auslesevorrichtung.

Der Bildträger weist einen elektronischen Speicher zur Speicherung von Kalibrierdaten des Bildträgers auf, wobei die Kalibrierdaten ein Maß für die Empfindlichkeit des Bildträgers für Röntgenstrahlung darstellen und bei der Verarbeitung von Bildsignalen, welche bei einem Auslesen der Röntgeninformation aus dem Bildträger erhalten werden, herangezogen werden können.

Durch die Speicherung der Kalibrierdaten in dem auf dem Bildträger befindlichen elektronischen Speicher kann eine zusätzliche Übermittlung der Kalibrierdaten über einen zusätzlichen Datenträger entfallen. Hierdurch wird außerdem eine Verwechslung einzelner Datenträger ausgeschlossen und damit die Zuverlässigkeit bei der Berücksichtigung unterschiedlicher Empfindlichkeiten der Bildträger erhöht.

Der elektronische Speicher ist vorzugsweise als integrierter elektronischer Schaltkreis mit einem nicht-flüchtigen Speicher ausgebildet, beispielsweise als ROM, PROM, EPROM oder EEPROM.

Vorzugsweise stellen die Kalibrierdaten des Bildträgers ein Maß für eine über eine Speicherleuchtstoff-Schicht des Bildträgers lokal veränderliche Empfindlichkeit des Bildträgers für Röntgenstrahlung dar. Dadurch werden nicht nur unterschiedliche Empfindlichkeiten einzelner Bildträger berücksichtigt, sonder auch individuell auftretende Variationen der Empfindlichkeit über die gesamte Fläche der Speicherleuchtstoff-Schicht eines einzelnen Bildträgers. Die Genauigkeit der Kalibrierung wird dadurch erhöht.

Die Kalibrierdaten, welche ein Maß für die lokal veränderliche Empfindlichkeit des Bildträgers für Röntgenstrahlung darstellen, sind vorzugsweise in einem zweidimensionalen Datenfeld im elektronischen Speicher des Bildträgers gespeichert. Hierdurch wird eine übersichtliche Korrelation einzelner Bereiche der Speicherleuchtstoff-Schicht mit deren jeweiliger Empfindlichkeit erreicht.

In einer weiteren Ausgestaltung ist vorgesehen, dass die Kalibrierdaten des Bildträgers die lokal veränderliche Empfindlichkeit des Bildträgers in einzelnen Bereichen des Bildträgers wiedergeben, wobei die einzelnen Bereiche den einzelnen Bildpunkten (Pixel) entsprechen, die bei einem Auslesen der im Bildträger gespeicherten Röntgeninformation erhalten werden. Hierdurch wird eine pixelgenaue Kalibrierung ermöglicht, die lokal unterschiedliche Empfindlichkeiten mit sehr hoher Genauigkeit berücksichtigt.

In einer alternativen Ausgestaltung kann aber auch vorgesehen sein, dass die Kalibrierdaten des Bildträgers die lokal veränderliche Empfindlichkeit des Bildträgers in einzelnen Bereichen des Bildträgers wiedergeben, wobei die einzelnen Bereiche größer sind als die einzelnen Bildpunkte (Pixel), die bei einem Auslesen der im Bildträger gespeicherten Röntgeninformation erhalten werden. Auf diese Weise kann der Speicherplatzbedarf für die Kalibrierdaten im elektronischen Speicher stark reduziert werden, wobei gleichzeitig lokal unterschiedliche Empfindlichkeiten dennoch mit einer hohen Genauigkeit berücksichtigt werden.

In einer weiteren Ausgestaltung der Erfindung sind die Kalibrierdaten des Bildträgers in einer Kalibrierungs-Datengruppe im elektronischen Speicher des Bildträgers gespeichert. Dadurch wird sowohl eine übersichtliche Aufteilung des Speicherinhalts erreicht als auch ein einfacherer Zugriff auf die Kalibrierdaten der Kalibrier-Datengruppe ermöglicht.

In einer weiteren vorteilhaften Ausgestaltung umfasst der Bildträger eine Bildplatte zur Speicherung der Röntgeninformation, wobei der elektronische Speicher vorzugsweise an der Bildplatte angebracht ist. Dadurch wird eine sichere Zuordnung der im elektronischen Speicher gespeicherten Kalibrierdaten zur zugehörigen Bildplatte gewährleistet.

In einer Ausführung dieser Variante ist vorgesehen, dass die Bildplatte eine Trägerschicht mit einer auf der Vorderseite der Trägerschicht befindlichen Speicherleuchtstoff-Schicht umfasst und der elektronische Speicher in einem Randbereich der Trägerschicht, insbesondere außerhalb der Speicherleuchtstoff-Schicht, und/oder an der der Vorderseite gegenüberliegenden Rückseite der Trägerschicht angebracht ist. Hierdurch wird eine Beeinträchtigung der Speichereigenschaften der Speicherleuchtstoff-Schicht durch den elektronischen Speicher vermieden.

In der Regel umfasst der Bildträger neben der Bildplatte eine Kassette, welche die Bildplatte unter Abschirmung von Umgebungslicht aufnehmen kann. Ist der elektronische Speicher zur kontaktlosen Datenübertragung ausgebildet, kann sein Speicherinhalt mit einem entsprechenden RF-Leser auch von der Außenseite der Kassette durch die Kassettenwand hindurch ausgelesen werden.

In einer alternativen Ausgestaltung der Erfindung ist der elektronische Speicher an der Kassette angebracht. Die Kassette muss in diesem Fall nicht geöffnet und die Bildplatte nicht entnommen werden, wenn der elektronische Speicher nur durch galvanische Kontaktierung ausgelesen werden kann.

Vorteilhafterweise sind die Kalibrierdaten im elektronischen Speicher des Bildträgers in komprimierter Form gespeichert. Hierdurch kann der Speicherplatzbedarf zum Teil stark reduziert werden. Als Datenkomprimierungsverfahren eignet sich beispielsweise JPEG.

Ein entsprechendes System zur Bearbeitung eines Bildträgers zur Speicherung von Röntgeninformation besteht aus mindestens einem Bildträger und mindestens einer Vorrichtung zur Bearbeitung des Bildträgers und/oder zur Weiterverarbeitung und/oder Wiedergabe der im Bildträger gespeicherten Röntgeninformation und zeichnet sich dadurch aus, dass der Bildträger einen elektronischen Speicher zur Speicherung von Kalibrierdaten des Bildträgers umfasst, welche ein Maß für die Empfindlichkeit des Bildträgers für Röntgenstrahlung darstellen und bei einer Verarbeitung von Bildsignalen, welche bei einem Auslesen der Röntgeninformation aus dem Bildträger erhalten werden, herangezogen werden können.

Hierbei ist eine Vorrichtung als Auslesevorrichtung ausgebildet, welche die im Bildträger gespeicherte Röntgeninformation auslesen und dabei entsprechende Bildsignale erzeugen kann und die erzeugten Bildsignale unter Heranziehung der im elektronischen Speicher gespeicherten Kalibrierdaten des Bildträgers verarbeiten kann. Vorzugsweise ist die Auslesevorrichtung mit einem entsprechenden Lesegerät ausgestattet, mit dem die im elektronischen Speicher des Bildträgers gespeicherten Kalibrierdaten des Bildträgers gelesen werden können.

Weitere Merkmale und Vorteile der Erfindung ergeben sich aus der folgenden Beschreibung bevorzugter Ausführungsformen und Anwendungsbeispiele, wobei Bezug auf die beigefügten Figuren genommen wird.

Es zeigen:

Fig. 1    ein System zur Bearbeitung eines Bildträgers für Röntgeninformation;

Fig. 2    ein erstes Beispiel einer Struktur der im integrierten Schaltkreis des Bildträgers gespeicherten Daten;

Fig. 3    eine schematische Darstellung einer ersten Variante des Datenflusses zwischen einzelne Komponenten des in Fig. 1 dargestellten Systems;

Fig. 4    eine schematische Darstellung einer zweiten Variante des Datenflusses zwischen einzelnen Komponenten des in Fig. 1 dargestellten Systems; und

Fig. 5    ein zweites Beispiel einer Struktur der im integrierten Schaltkreis des Bildträgers gespeicherten Daten.

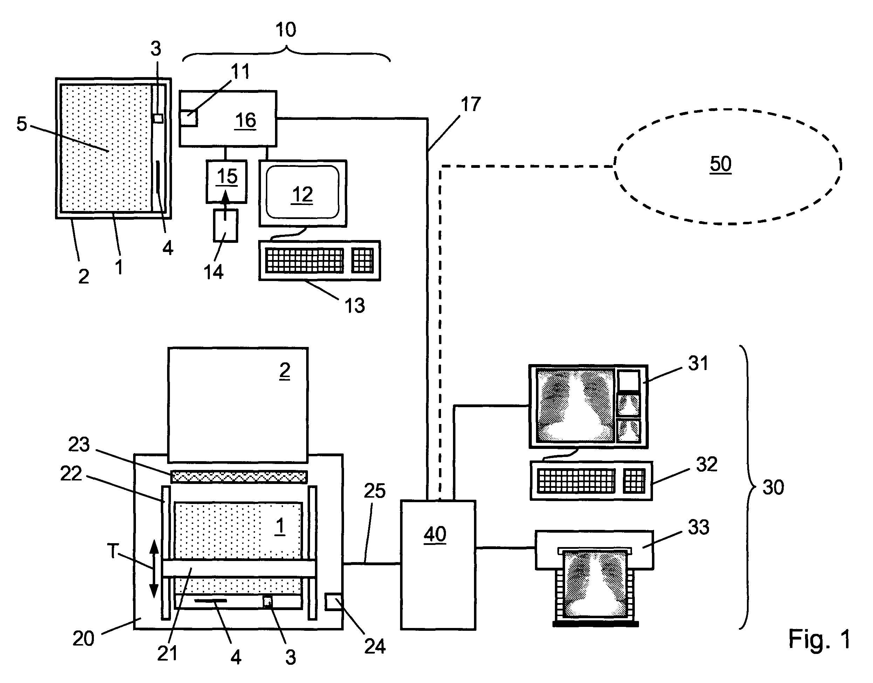

Fig. 1 zeigt ein System zur Bearbeitung eines Bildträgers für Röntgeninformation. Das System umfasst eine Identifikationsstation 10, welche im Folgenden auch als ID-Station bezeichnet wird, eine Auslesevorrichtung 20, eine Wiedergabevorrichtung 30 sowie einen Zentralspeicher 40.

Der Bildträger für Röntgeninformation besteht aus einer Kassette 2 mit einer darin befindlichen Bildplatte 1. Die Bildplatte 1 umfasst eine Trägerschicht mit einer darauf aufgebrachten Speicherleuchtstoff-Schicht 5. Die Speicherleuchtstoff-Schicht 5 weist vorzugsweise einen Speicherleuchtstoff auf der Basis von BaFBr:Eu oder CsBr:Eu auf.

Die Bildplatte 1 ist mit einem integrierten Schaltkreis 3 versehen, der einen Schreib-Lese-Speicher umfasst, in welchen Daten geschrieben und aus welchem Daten gelesen werden können. Um die Speicherfunktion der Speicherleuchtstoff-Schicht 5 nicht zu beeinträchtigen, wird der integrierte Schaltkreis 3 vorzugsweise an der Rückseite oder - wie im dargestellten Beispiel - im Randbereich der Bildplatte 1 angeordnet.

Alternativ oder zusätzlich kann der integrierter Schaltkreis 3 an der Kassette 2 angebracht sein. Die folgenden Ausführungen, die sich auf einen an der Bildplatte 1 befindlichen integrierten Schaltkreis 3 beziehen, gelten auch für diese Alternative entsprechend.

Nach einer Röntgenaufnahme wird die in der Kassette 2 befindliche Bildplatte 1, in deren Speicherleuchtstoff-Schicht 5 Röntgeninformation gespeichert ist, zur ID-Station 10 gebracht, um Daten aus dem und/oder in den integrierten Schaltkreis 3 zu lesen bzw. zu schreiben. Die Datenübertragung zwischen dem integrierten Schaltkreis 3 und der ID-Station 10 erfolgt vorzugsweise kontaktlos. Hierdurch kann eine exakte Positionierung der Bildplatte 1 und des Schaltkreises 3 relativ zur ID-Station 10, wie sie bei einer kontaktbehafteten Datenübertragung erforderlich wäre, entfallen. Die kontaktlose Datenübertragung erfolgt vorzugsweise mittels Radiofrequenz-Wellen (RF-Wellen). Zu diesem Zweck weist die ID-Station 10 eine entsprechende erste Schreib-Lese-Einrichtung 11 mit einem RF-Sender und einem RF-Empfänger auf.

Der integrierte Schaltkreis 3 ist vorzugsweise in Form eines sogenannten RF-Etiketts, welches auch als RF-Tag bezeichnet wird, an der Bildplatte 1 angebracht, beispielsweise aufgeklebt. Ein solches RF-Tag umfasst neben dem integrierten Schaltkreis 3 eine als Transponderspule ausgebildete Antenne. Statt als RF-Tag kann der integrierte Schaltkreis 3 auch in Form einer Chipkarte, bei welcher ein RF-Tag in einen kartenförmigen Plastikkörper eingebracht ist, an der Bildplatte 1 angebracht sein. Die Chipkarte wird vorzugsweise lösbar an der Bildplatte 1 befestigt, z.B. mittels einer einfachen Steckverbindung im Randbereich der Bildplatte 1. Zur zusätzlichen Sicherung der Chipkarte kann ein Arretiermechanismus vorgesehen sein, z.B. ein Schnappmechanismus, bei welchem die Chipkarte durch Zuschnappen arretiert wird. Diese Befestigung der Chipkarte an der Bildplatte 1 ist einerseits einfach und sicher und vereinfacht andererseits den - beispielsweise im Falle eines Defekts des integrierten Schaltkreises 3 erforderlichen - Austausch der Chipkarte gegen eine andere.

Die Bildplatte 1 ist mit einer mit dem bloßen Auge lesbaren Markierung 4 versehen, welche zumindest einen Teil der im Speicher des integrierten Schaltkreises 3 gespeicherten Daten, insbesondere für die Bildplatte 1 spezifische Daten, repräsentiert und vorzugsweise als alphanumerische Zeichenfolge ausgebildet ist. Hierdurch sind bei einem Defekt des integrierten Schaltkreises 3, bei dem die darin gespeicherten Daten im Allgemeinen nicht mehr ausgelesen werden können, die in der Markierung 4 enthaltenen Daten weiterhin zugänglich. Der defekte integrierte Schaltkreis 3 kann in diesem Fall gegen einen neuen ausgetauscht werden, in welchen dann die in der Markierung 4 enthaltenen Daten geschrieben werden. Hierzu werden die Daten der Markierung 4 von einem Bediener gelesen, an der ID-Station 10 eingegeben und schließlich in den neuen integrierten Schaltkreis geschrieben. Mit Hilfe der Markierung 4 wird damit auf einfache Weise sichergestellt, dass der im integrierten Schaltkreis 3 gespeicherte und in der Markierung 3 enthaltene Teil der Daten auch bei einem Defekt des Schaltkreises 3 nicht verlorengeht.

Alternativ oder zusätzlich kann eine derart ausgestaltete Markierung 4 auch an der Kassette 2 angebracht sein. Das Vorgehen bei einem Defekt des integrierten Schaltkreises erfolgt in analoger Weise.

Die ID-Station 10 umfasst eine oder mehrere Eingabeeinrichtungen zur Eingabe von Daten, welche beispielsweise für einen zu untersuchenden Patienten, das Auslesen der Bildplatte 1 oder die Weiterverarbeitung von aus der Bildplatte 1 ausgelesenen Bilddaten spezifisch bzw. erforderlich sind. Im dargestellten Beispiel umfassen die Eingabeeinrichtungen eine Tastatur 13 mit Anzeigeeinheit 12, wie z. B. einen Monitor, sowie einen Kartenleser 15 für eine Karte 14, auf welcher sich einzugebende Daten befinden. Insbesondere handelt es sich bei der Karte 14 um eine Chipkarte, beispielsweise eine Krankenversicherungskarte, auf welcher patientenspezifische Daten, wie z.B. Name, Adresse, Geburtsdatum und Versicherungsnummer eines Patienten, gespeichert sind. Der Kartenleser 15 liest diese Daten aus der Karte 14 aus und übergibt sie an einen Zwischenspeicher 16 der ID-Station 10. Auch die über die Tastatur 13 eingegebenen Daten werden an den Zwischenspeicher 16 übergeben. Bei der Anzeigeeinheit 12 kann es sich vorzugsweise auch um einen sog. Touchscreen handeln, bei welchem angezeigte Funktionen und/oder Daten durch Berührung der entsprechenden Bereiche der Anzeige ausgewählt werden können.

Vom Zwischenspeicher 16 aus werden die eingegebenen Daten an die erste Schreib-Lese-Einrichtung 11 übergeben und in den Speicher des integrierten Schaltkreises 3 auf der Bildplatte 1 geschrieben.

Anstelle der eingegebenen Daten selbst kann ein diesen Daten zugeordneter Referenzcode in den Speicher des integrierten Schaltkreises 3 geschrieben werden, während die eingegebenen Daten zusammen mit dem ihnen zugeordneten Referenzcode im Zwischenspeicher 16 und/oder im Zentralspeicher 40 gespeichert werden. Auf dieses Verfahren wird weiter unten noch näher eingegangen.

Anschließend wird die Kassette 2 mit der darin befindlichen Bildplatte 1 zur Auslesevorrichtung 20 gebracht, wo die Kassette 2 automatisch geöffnet, die Bildplatte 1 entnommen und in das Innere der Auslesevorrichtung 20 eingezogen wird. In der hier dargestellten Position ist die Bildplatte 1 bereits vollständig aus der Kassette herausgezogen und im Innern der Auslesevorrichtung 20 arretiert. In dieser Position wird sie von einem Scanner 21, welcher mit einem geeigneten Transportmechanismus 22 in Transportrichtung T über die Bildplatte 1 bewegt wird, ausgelesen.

Der Scanner 21 ist vorzugsweise als sog. Zeilenscanner ausgebildet, welcher eine zeilenförmige Stimulationslichtquelle, vorzugsweise mit in einer Reihe angeordneten Laserdioden, und einen zeilenförmigen Detektor, vorzugsweise ein lineares CCD-Array, aufweist. Während der Bewegung des Scanners 21 über die Bildplatte 1 wird diese mit dem Licht der Stimulationslichtquelle zeilenweise bestrahlt, wobei in der Speicherleuchtstoff-Schicht 5 Emissionslicht angeregt wird, dessen Intensität der in der Speicherleuchtstoff-Schicht 5 gespeicherten Röntgeninformation entspricht. Das Emissionslicht wird mit dem zeilenförmigen Detektor erfasst und in entsprechende Bildsignale umgewandelt. Durch die kontinuierliche Bewegung des Scanners 21 in Transportrichtung T über die Bildplatte 1 wird die Speicherleuchtstoff-Schicht 5 sukzessive zeilenweise ausgelesen, wobei ein zweidimensionales Abbild der gespeicherten Röntgeninformation erhalten wird.

Alternativ kann der Scanner 21 auch als sog. Flying Spot-Scanner ausgebildet sein, bei welchem ein einzelner Laserstrahl durch einen rotierenden Polygonspiegel auf die Speicherleuchtstoff-Schicht 5 gelenkt wird, wodurch diese entlang einzelner Zeilen punktweise abgetastet wird.

Nachdem die Bildplatte 1 vollständig ausgelesen worden ist, wird diese wieder automatisch zurück in die Kassette 2 befördert. Dabei passiert sie eine Löscheinrichtung 23, an welcher etwaige restliche Röntgeninformationen in der Bildplatte 1 durch Bestrahlung der Speicherleuchtstoff-Schicht 5 mit Löschstrahlung gelöscht werden. Die Löscheinrichtung 23 umfasst hierzu eine Strahlungsquelle mit einem - verglichen mit der Stimulationslichtquelle - breitbandigerem Spektrum und einen geeigneten Reflektor zur Reflexion von Löschstrahlung auf die Speicherleuchtstoff-Schicht 5 der Bildplatte 1.

Die Auslesevorrichtung 20 umfasst eine zweite Schreib-Lese-Einrichtung 24, mit welcher Daten aus dem bzw. in den integrierten Schaltkreis 3 gelesen bzw. geschrieben werden können. Wie die erste Schreib-Lese-Einrichtung 11 der ID-Station 10 ist auch die zweite Schreib-Lese-Einrichtung 24 vorzugsweise zur kontaktlosen Datenübertragung, insbesondere mittels RF-Wellen, ausgebildet. Die mit der zweiten Schreib-Lese-Einrichtung 24 aus dem Speicher des integrierten Schaltkreises 3 ausgelesenen Daten werden insbesondere zur Steuerung des Auslesens der Bildplatte 1 mit dem Scanner 21 und/oder zur Steuerung des Löschens der Bildplatte 1 mit der Löscheinrichtung 23 herangezogen. Darüber hinaus können mit der zweiten Schreib-Lese-Einrichtung 24 Daten in den integrierten Schaltkreis 3 geschrieben werden, um vorzugsweise Daten zum Bearbeitungsstatus der Bildplatte 1 - beispielsweise, ob die Bildplatte 1 bereits ausgelesen bzw. gelöscht worden ist - zu aktualisieren.

Beim zeilenweisen Auslesen der Bildplatte 1 mit dem Scanner 21 werden Bilddaten erzeugt, welche die in der Bildplatte 1 gespeicherte Röntgeninformation repräsentieren. Diese Bilddaten werden über einen ersten Datenbus 25, insbesondere einen seriellen Datenbus, wie z.B. einen sog. Fire Wire, an den Zentralspeicher 40 übertragen und dort gespeichert.

Die im Zentralspeicher 40 gespeicherten Bilddaten können dann in der Wiedergabevorrichtung 30 weiterverarbeitet und/oder wiedergegeben werden. Zu diesem Zweck umfasst die Wiedergabevorrichtung 30 einen mittels einer Tastatur 32 a n-steuerbaren Monitor 31. Alternativ oder zusätzlich kann auch ein Hardcopygerät 33, beispielsweise ein Laserdrucker, zur Ausgabe der Bilddaten vorgesehen sein.

Der Zentralspeicher 40 kann optional an einem Netzwerk 50, insbesondere an einem lokalen Netzwerk (LAN), angeschlossen sein. Hierdurch wird ermöglicht, dass von anderen Systemen aus, insbesondere von anderen ID-Stationen und/oder Wiedergabevorrichtungen aus, auf die im Zentralspeicher 40 abgespeicherten Daten bzw. Bilddaten zugegriffen werden kann.

Der Zentralspeicher 40 kann als separate zentrale Einheit ausgebildet sein, welche z.B. in einem zentralen File-Server integriert sein kann. Alternativ kann der Zentralspeicher 40 aber auch integraler Bestandteil der ID-Station 10, der Aus|esevorrichtung 20 oder der Wiedergabevorrichtung 30 sein.

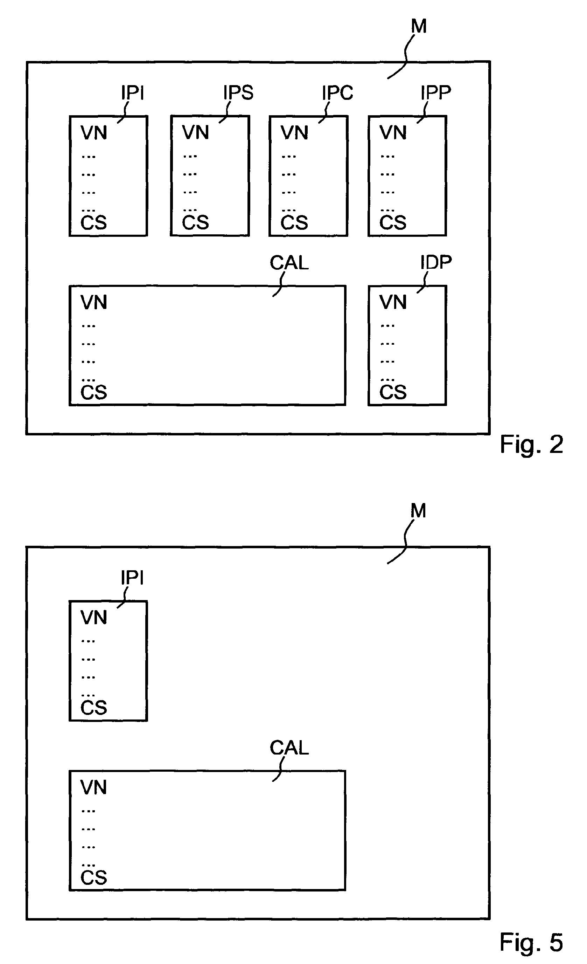

Fig. 2 zeigt ein erstes Beispiel einer Struktur der im Speicher M des integrierten Schaltkreises 3 des Bildträgers gespeicherten Daten. Anhand dieser Struktur wird nachfolgend die Datenübertragung innerhalb des in Fig. 1 beschriebenen Systems näher erläutert. Es ist zu beachten, dass die hier gewählte Darstellung die Struktur der Daten nur stark schematisiert wiedergibt und nicht auf eine bestimmte räumliche Anordnung oder eine bestimmte Reihenfolge der Daten im Speicher M des integrierten Schaltkreises 3 beschränkt ist.

Die im Speicher M gespeicherten Daten sind in unterschiedlichen Datengruppen IPI, IPS, IPC, IPP, CAL und IDP zusammengefasst und dort jeweils als ASCII-String abgelegt. Jeder Datengruppe ist eine Versionsnummer VN sowie eine Prüfsumme CS zugeordnet.

Die am Beginn jeder Datengruppe stehende Versionsnummer VN gibt an, in welcher Datenstruktur die Daten einer Datengruppe abgelegt sind. Hierdurch wird die Möglichkeit eröffnet, unterschiedliche Datenstrukturen ein und derselben Datengruppe vorzusehen. Dies ist immer dann erforderlich, wenn andere oder zusätzliche Daten im Speicher M abgelegt werden sollen, beispielsweise bei einem neuen Bildplattentyp oder einer neuen Art der Weiterverarbeitung der Bilddaten. Anhand der Versionsnummer VN einer Datengruppe können die einzelnen Komponenten des Systems, z.B. die ID-Station 10, die Auslesevorrichtung 20 bzw. die Wiedergabevorrichtung 30, erkennen, welche Daten in der jeweiligen Datengruppe enthalten sind und in welcher Datenlänge, Reihenfolge usw. diese Daten vorliegen.

Die am Ende jeder Datengruppe stehende Prüfsumme CS wird aus den einzelnen Daten dieser Datengruppe abgeleitet. Anhand der jeweiligen Prüfsumme CS kann festgestellt werden, ob die Daten dieser Datengruppe fehlerfrei gespeichert bzw. gelesen worden sind. Als Prüfsumme CS dient vorzugsweise der Rest der Division der Summe Σ der Zahlenwerte aller in dieser Datengruppe enthaltenen Bytes durch 256, also

$$CS = \Sigma \ Modulo \ 256.$$

Im Folgenden wird die Struktur und die Funktion der einzelnen Datengruppen IPI, IPS, IPC, IPP, CAL und IDP näher erläutert.

Eine Bildträger-Datengruppe IPI enthält spezifische Daten der Bildplatte 1 und/oder der Kassette 2. Diese Daten werden bei der Herstellung der Bildplatte 1 bzw. der Kassette 2 im Speicher M gespeichert und bleiben bei der Bearbeitung der Bildplatte 1 bzw. der Kassette 2 an der ID-Station 10 und/oder in der Auslesevorrichtung 20 unverändert, d.h. an der ID-Station 10 bzw. in der Auslesevorrichtung 20 werden die in der Bildträger-Datengruppe IPI gespeicherten Daten ausschließlich gelesen. Neben der Versionsnummer VN und der Prüfsumme CS umfasst die Bildträger-Datengruppe IPI folgende Daten:

- Initialisierungsdatum: entspricht dem Datum, an welchem die Daten der Bildträger-Datengruppe IPI in den Speicher M der Bildplatte 1 geschrieben wurden;
- Bildplatten-Seriennummer: dient zur eindeutigen Identifizierung der Bildplatte 1 und ist vorzugsweise aus einem die jeweilige Produktcharge der Bildplatte 1 identifizierenden Code und einer laufenden Nummer zusammengesetzt;
- Bildplattengröße;
- Größe einer auszulesenden Fläche auf der Bildplatte 1;
- Bildplattentyp: z.B. Bildplatte auf der Basis von pulverförmigen oder nadelförmigen Speicherleuchtstoffen, wie z.B. sogenannte Powder-IP bzw. Needle-IP;
- Bildplatten-Empfindlichkeit;
- Bildplatten-Löscheigenschaft: z.B. als Maß für die Dauer und/oder Intensität des von einer Löscheinrichtung 23 abgegebenen Lichts.

Vorzugsweise wird der Inhalt der Bildträger-Datengruppe IPI zusätzlich als visuell lesbare Markierung 4 (siehe Fig. 1) auf die Bildplatte 1 und/oder die Kassette 2 aufgebracht. Dies hat den Vorteil, dass die für die Bildplatte 1 bzw. Kassette 2 spezifischen und für deren Bearbeitung erforderlichen Daten der Bildträger-Datengruppe IPI auch dann nicht verlorengehen können, wenn der integrierte Schaltkreis 3 defekt ist und nicht mehr ausgelesen werden kann. In einem solchen Fall wird der defekte integrierte Schaltkreis 3 einfach gegen einen neuen integrierten Schaltkreis ausgetauscht, welcher dann mit den der Markierung 4 entnommenen Daten beschrieben wird. Eine solche Markierung 4 kann beispielsweise wie folgt lauten:

$$301 - 6KBQMF0001 - 20030702 - 1 - 1 - 0 - 1000 - 1000 - 123$$

Die ersten drei (301) sowie die letzten drei (123) Zeichen beinhalten die Versionsnummer VN bzw. die Prüfsumme CS. Nach der Versionsnummer VN folgen die Bildplatten-Seriennummer (6KBQMF0001), das Initialisierungsdatum (20030702), Werte für die Bildplattengröße (1), die Größe (1) der auszulesenden Fläche der Bildplatte sowie den Bildplattentyp (0), die Bildplatten-Empfindlichkeit (1000) und schließlich die Bildplatten-Löscheigenschaft (1000).

Die der Markierung 4 entnommenen Daten können an der ID-Station 10 auf einfache Weise in den Speicher des neuen integrierten Schaltkreises geschrieben werden, ohne dass eine neue Initialisierung beim Hersteller der Bildplatte 1 bzw. Kassette 2 erforderlich wäre. Im einfachsten Fall werden die Daten der Markierung 4 von einem Bediener gelesen, über die Tastatur 13 der ID-Station 10 eingegeben und anschließend in den Speicher des neuen integrierten Schaltkreises geschrieben.

Die oben beschriebene visuell, d.h. mit dem menschlichen Auge, lesbare Markierung 4 stellt eine besonders einfache Möglichkeit zur zusätzlichen Sicherung von im integrierten Schaltkreis 3 gespeicherten Daten dar. Selbstverständlich kann anstelle einer visuell lesbaren Markierung alternativ oder zusätzlich eine maschinell lesbare Markierung (nicht dargestellt) vorgesehen sein, beispielsweise in Form eines Barcodes oder Magnetstreifens. Zur Eingabe der in der maschinell lesbaren Markierung enthaltenen Daten ist dann ein entsprechendes Barcode- bzw. Magnetstreifenlesegerät erforderlich, das die maschinell eingelesenen Daten an die ID-Station 10 weiterleitet, wo diese dann in den neuen integrierten Schaltkreis geschrieben werden können.

Die Daten einer Zustands-Datengruppe IPS betreffen den jeweiligen Bearbeitungszustand der Bildplatte 1 bzw. der Kassette 2 und werden sowohl in der ID-Station 10 als auch in der Auslesevorrichtung 20 geändert. Neben der Versionsnummer VN und der Prüfsumme CS umfasst diese Datengruppe folgende Daten:

- Bildplattenstatus: z.B. ob die Bildplatte 1 bereits initialisiert ist (d.h. Daten der Bildträger-Datengruppe IPI im Speicher M gespeichert sind) und/oder ein Röntgenbild bereits aufgenommen, gelöscht bzw. noch zu löschen ist;
- Bildplattenzyklen: Gesamtzahl von Röntgenaufnahmen mit dieser Bildplatte 1. Diese Gesamtzahl wird nach jedem Auslesevorgang oder Löschvorgang in der Auslesevorrichtung 20 um den Wert 1 erhöht;
- Auslesen unter Biegung: Anzahl der Bildplattenzyklen, in welchen die Bildplatte 1 während des Auslesevorgangs gekrümmt wird. Diese Zahl ist ein Maß für den Abnutzungs- und/oder Beschädigungsgrad einer Bildplatte 1. Dieser Eintrag kann beispielsweise bei Systemen entfallen, in welchen die Bildplatte während des Auslesens auf einer festen, ebenen Unterlage aufliegt und folglich nicht gebogen wird.

Eine Steuerungs-Datengruppe IPC umfasst alle für eine Röntgenaufnahme spezifischen Daten, die für das Auslesen der Bildplatte 1 in der Auslesevorrichtung 20 erforderlich sind. Die Auslesevorrichtung 20 greift dabei ausschließlich lesend auf die in der Steuerungs-Datengruppe IPC gespeicherten Daten zu. Neben der Versionsnummer VN und der Prüfsumme CS umfasst die Steuerungs-Datengruppe IPC folgende Daten:

- Scannerempfindlichkeit: gibt die im Scanner 21 beim Auslesen der Bildplatte 1 einzustellende Empfindlichkeit an und ist abhängig von der Röntgendosis in Bereichen der Bildplatte 1, welche diagnostische Informationen enthalten;
- Röntgendosis: entspricht der maximalen Röntgendosis während einer Röntgenaufnahme und stellt ein Maß für die einzustellende Intensität der Löscheinrichtung 23 beim Löschen der Bildplatte 1 dar;
- Betriebsmodus: z.B. Weglassen oder Auslesen von Röntgeninformation aus bestimmten Randbereichen der Bildplatte 1.

In einer Verarbeitungs-Datengruppe IPP sind Daten gespeichert, die für eine Weiterverarbeitung von aus der Röntgeninformation der Bildplatte 1 gewonnenen Bilddaten erforderlich sind. Auf diese Daten wird ausschließlich von der Wiedergabevorrichtung 30, welche einen entsprechenden Bildprozessor zur Weiterverarbeitung der Bilddaten umfasst, zugegriffen. Der Auslesevorgang in der Auslesevorrichtung 20 wird durch diese Daten nicht beeinflusst.

Vorzugsweise werden die Daten der Verarbeitungs-Datengruppe IPP mit der zweiten Schreib-Lese-Einrichtung 24 in der Auslesevorrichtung 20 gelesen und zusammen mit den von der Bildplatte 1 gewonnenen Bilddaten im Zentralspeicher 40 abgelegt. Die Wiedergabevorrichtung 30 kann dann auf einfache Weise sowohl auf die zu verarbeitenden Bilddaten als auch auf die hierfür erforderlichen Daten der Verarbeitungs-Datengruppe IPP zugreifen.

In einer bevorzugten Ausgestaltung ist vorgesehen, die für die Weiterverarbeitung der Bilddaten erforderlichen Daten der Verarbeitungs-Datengruppe IPP in der ID-Station 10 einzugeben und diesen eindeutige Referenzcodes (IPP-Ref) zuzuordnen, die dann anstelle der Daten für die Weiterverarbeitung in den Speicher M geschrieben werden. Die Daten für die Weiterverarbeitung selbst werden zusammen mit den Referenzcodes (IPP-Ref) im Zentralspeicher 40 gespeichert. Die Wiedergabevorrichtung 30, welche die im Zentralspeicher 40 gespeicherten Daten für die Weiterverarbeitung benötigt, kann dann anhand der mit den Bilddaten von der Auslesevorrichtung 20 übertragenen Referenzcodes (IPP-Ref) auf die in der im Zentralspeicher 40 gespeicherten Daten für die Weiterverarbeitung zugreifen. Neben der Versionsnummer VN und der Prüfsumme CS umfasst die Verarbeitungs-Datengruppe IPP in diesem Fall folgende Daten:

- Referenzcode für die Art der durchzuführenden Weiterverarbeitung;
- Referenzcode für Aufnahme- und Patientendaten;
- Eindeutige Kennzeichnung des Zentralspeichers, auf welchen die Referenzcodes verweisen, wie z.B. der Name des Zentralspeichers in einem Netzwerk.

Eine Kalibrierungs-Datengruppe CAL umfasst Kalibrierdaten der Bildplatte 1. Die Kalibrierdaten liegen vorzugsweise als zweidimensionales Datenfeld vor, welches die unterschiedliche lokale Empfindlichkeit der Bildplatte 1 für Röntgenstrahlung widerspiegelt. Die Daten der Kalibrierungs-Datengruppe CAL werden vorzugsweise zusammen mit den Daten der Bildträger-Datengruppe IPI bei der Herstellung und Initialisierung der Bildplatte 1 bzw. der Kassette 2 im Speicher M des integrierten Schaltkreises 3 gespeichert und während der Bearbeitung der Bildplatte 1 bzw. der Kassette 2 nicht geändert. Neben der Versionsnummer VN und der Prüfsumme CS umfasst die Kalibrierungs-Datengruppe CAL im Einzelnen folgende Daten:

- Anzahl der Spalten des zweidimensionalen Datenfeldes der Kalibrierdaten;
- Anzahl der Zeilen des zweidimensionalen Datenfeldes der Kalibrierdaten;
- zweidimensionales Datenfeld der Kalibrierdaten.

Die Kalibrierdaten der Bildplatte 1 werden in der Auslesevorrichtung 20 zu einer Vorauswertung der beim Auslesen der Bildplatte 1 mittels Scanner 21 erhaltenen Bildsignale herangezogen, um lokal unterschiedliche Empfindlichkeiten der Bildplatte 1 für Röntgenstrahlung zu berücksichtigen. Die Kalibrierdaten werden hierzu in der Auslesevorrichtung 20 aus dem integrierten Schaltkreis 3 gelesen und der Vorverarbeitung der Bildsignale zugeführt.

Vorzugsweise werden einmal ausgelesene Kalibrierdaten in einem Speicher (nicht dargestellt) der Auslesevorrichtung 20 gespeichert oder über den ersten Datenbus 25 an den Zentralspeicher 40 übertragen und dort gespeichert. Wird dieselbe Bildplatte 1 mit einer anderen Röntgenaufnahme dann erneut einem Auslesevorgang in derselben Auslesevorrichtung 20 unterzogen, so kann diese Auslesevorrichtung 20 direkt auf den Speicher der Auslesevorrichtung 20 bzw. den Zentralspeicher 40 zugreifen und die für die Vorverarbeitung der Bildsignale erforderlichen Kalibrierdaten der Bildplatte 1 abrufen. Die Kalibrierdaten stehen dadurch in kürzerer Zeit für die Vorverarbeitung zur Verfügung als bei einem erneuten Auslesen des zweidimensionalen Datensatzes aus dem integrierten Schaltkreis 3. Auch etwaige Übertragungsfehler beim Auslesen des Datensatzes aus dem integrierten Schaltkreis können dadurch vermieden werden.

Alternativ können die Kalibrierdaten bereits an der ID-Station 10 aus dem integrierten Schaltkreis 3 ausgelesen, über einen zweiten Datenbus 17 an den Zentralspeicher 40 übertragen und dort gespeichert werden. Ein Auslesen und Übertragen der Kalibrierdaten zum Zentralspeicher 40 während des ersten Auslesevorgangs in der Auslesevorrichtung 20 kann dann entfallen. Der zweite Datenbus 17 ist vorzugsweise ebenfalls als serieller Datenbus, z.B. als RS-232-Datenbus, ausgebildet.

Prinzipiell ist es möglich, das zweidimensionale Datenfeld der Kalibrierdaten der Bildplatte 1 bei jedem Auslesevorgang in der Auslesevorrichtung 20 aus dem integrierten Schaltkreis 3 von Neuem auszulesen. In diesem Fall kann gegebenenfalls auf eine permanente Verbindung zwischen der Auslesevorrichtung 20 und dem Zentralspeicher 40 ganz verzichtet werden oder zumindest der Datenfluss zwischen den einzelnen Komponenten des Systems, insbesondere zwischen Auslesevorrichtung 20 und Zentralspeicher 40 besonders niedrig gehalten werden.

Neben den Kalibrierdaten der Bildplatte 1 werden im Zentralspeicher 40 oder im Speicher der Auslesevorrichtung 20 auch Kalibrierdaten des Scanners 21 abg e-legt, welche lokale Unterschiede in der Empfindlichkeit des Scanners 21 widerspiegeln und vorzugsweise zusammen mit den Kalibrierdaten der Bildplatte 1 zur Vorverarbeitung der Bildsignale herangezogen werden. Aus der Vorverarbeitung der Bildsignale werden schließlich Bilddaten des in der Bildplatte 1 gespeicherten Röntgenbildes erhalten, aus welchen der Einfluss lokal unterschiedlicher Empfindlichkeiten der Bildplatte 1 und des Scanners 21 eliminiert worden ist.

Typischerweise weist das zweidimensionale Datenfeld der Kalibrierdaten der Bildplatte 1 zwischen etwa 30 und 40 Spalten und zwischen etwa 40 und 50 Zeilen auf, wobei die Länge der jeweiligen Daten jeweils zwei Bytes beträgt. Die für ein solches zweidimensionales Datenfeld zu reservierende Speichergröße beträgt demnach für ein Datenfeld, welches vorzugsweise 35 Spalten und 43 Zeilen aufweist, 2 x 35 x 43 = 3010 Bytes.

In dem beschriebenen Beispiel geben die Kalibrierdaten die Empfindlichkeit von einzelnen Bereichen der Speicherleuchtstoff-Schicht 5 wieder, die größer sind als die einzelnen, beim zeilenweisen Auslesen der Bildplatte 1 erhaltenen Bildpunkte (Pixel). Auf diese Weise kann der Speicherplatzbedarf für die Kalibrierdaten im integrierten Schaltkreis 3 stark reduziert werden, wobei gleichzeitig lokal unterschiedliche Empfindlichkeiten mit einer hohen Genauigkeit berücksichtigt werden.

Wenn ausreichend Speicherplatz im integrierten Schaltkreis 3 vorhanden ist, können die Kalibrierdaten aber auch die Empfindlichkeit der Bildplatte 1 in einzelnen Bereichen der Bildplatte wiedergeben, die den einzelnen Bildpunkten entsprechen. Hierdurch wird eine pixelgenaue Kalibrierung ermöglicht, die lokal unterschiedliche Empfindlichkeiten mit noch höherer Genauigkeit berücksichtigt.

Um möglichst wenig Speicherplatz im Speicher M des integrierten Schaltkreises 3 zu beanspruchen, werden die Kalibrierdaten der Bildplatte 1 vorteilhafterweise einem Komprimierungsverfahren unterzogen, bevor diese im integrierten Schaltkreis 3 abgelegt werden.

In einer Patienten-Datengruppe IDP sind Daten gespeichert, welche für einen zu untersuchenden Patienten spezifisch sind. Neben der Versionsnummer VN und der Prüfsumme CS umfasst die Patienten-Datengruppe IDP beispielsweise folgende Daten:

- Name des Patienten;
- Geburtsdatum des Patienten;
- Geschlecht des Patienten.

Vorzugsweise wird anstelle der oder zusätzlich zu den genannten patientenspezifischen Daten der Patienten-Datengruppe IDP ein Referenzcode IDP-Ref in den Speicher M geschrieben, welcher eine Referenz auf die Daten der Patienten-Datengruppe IDP darstellt, die im Zentralspeicher 40 abgelegt sind. Der Referenzcode IDP-Ref, welcher auch als Patienten-Identifikationscode bezeichnet wird und eine eindeutige Identifikation des Patienten ermöglicht, wird mit der ersten Schreib-Lese-Einrichtung 11 der ID-Station 10 in den Speicher M des integrierten Schaltkreises 3 geschrieben und zusammen mit den Daten der Patienten-Datengruppe IDP im Zwischenspeicher 16 der ID-Station 10 und/oder im Zentralspeicher 40 gespeichert.

Anhand des Patienten-Identifikationscodes IDP-Ref kann dann auf die im Zwischenspeicher 16 bzw. Zentralspeicher 40 gespeicherten patientenspezifischen Daten zugegriffen werden. Der Patienten-Identifikationscode IDP-Ref wird hierzu entweder in der ID-Station 10 bzw. in der Auslesevorrichtung 20 aus dem integrierten Schaltkreis 3 gelesen und an die Wiedergabevorrichtung 30 übertragen oder zusammen mit den aus der Bildplatte 1 ausgelesenen Bilddaten an die Wiedergabevorrichtung 30 übertragen, welche dann anhand des Patienten-Identifikationscodes IDP-Ref auf die entsprechenden patientenspezifischen Daten im Zwischenspeicher 16 bzw. Zentralspeicher 40 zugreifen kann.

Prinzipiell kann die Patienten-Datengruppe IDP aber auch entfallen, wenn in der Verarbeitungs-Datengruppe IPP - wie oben bereits beschrieben - bereits Referenzcodes für Patientendaten vorgesehen sind, welche u.a. auf die entsprechenden Patientendaten, wie z.B. Name und/oder Geburtsdatum und/oder Geschlecht des Patienten, im Zentralspeicher 40 verweisen.

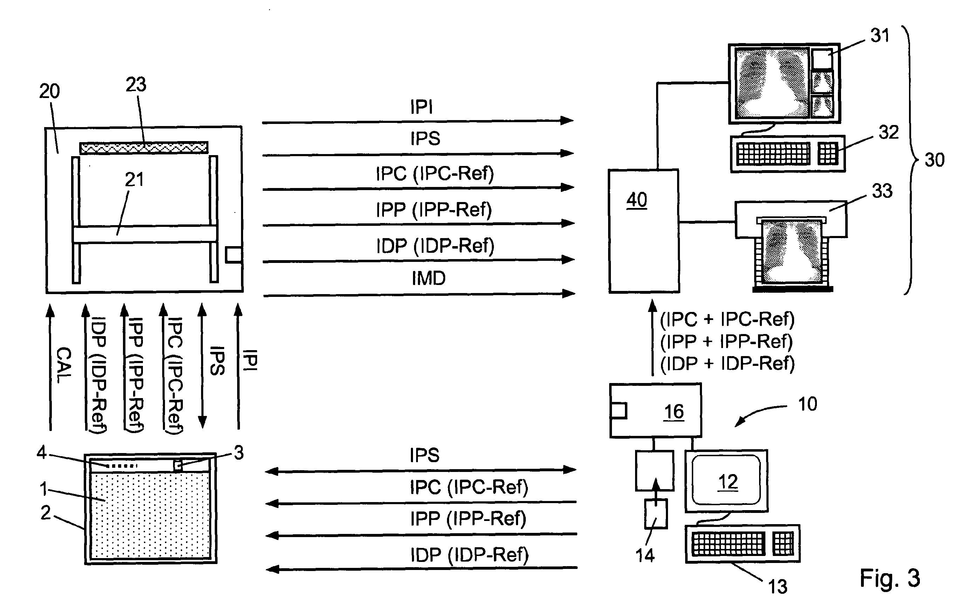

Fig. 3 zeigt eine schematische Darstellung einer ersten Variante des Datenflusses zwischen den einzelnen Komponenten des in Fig. 1 dargestellten Systems. Der Datenfluss wird nachfolgend anhand eines kompletten Radiographievorgangs näher erläutert.

Unmittelbar nach einer Röntgenaufnahme eines Patienten wird die Kassette 2 mit der darin befindlichen Bildplatte 1 zur ID-Station 10 gebracht. An der ID-Station 10 werden folgende Schritte durchgeführt:

a) Lesen von Daten der Zustands-Datengruppe IPS zum Bearbeitungsstatus der Bildplatte 1 aus dem Speicher M des integrierten Schaltkreises 3. Prüfen, ob der Bearbeitungsstatus auf "Bildplatte initialisiert" oder "Bildplatte gelöscht" gesetzt ist. Ist die Bildplatte nicht initialisiert oder nicht gelöscht, muss der Bediener zwischen zwei Alternativen wählen, nämlich einem Überschreiben der Daten oder einem Abbruch der Bearbeitung.

b) Eingabe von patientenspezifischen Daten der Patienten-Datengruppe IDP mittels Tastatur 13 und/oder Anzeigeeinheit 12 und/oder Karte 14.

c) Eingabe von für das Auslesen der Bildplatte 1 in der Auslesevorrichtung 20 erforderlichen Daten der Steuerungs-Datengruppe IPC mittels Tastatur 13 und/oder Anzeigeeinheit 12.

d) Eingabe von für die Weiterverarbeitung von Bilddaten in der Wiedergabevorrichtung 30 erforderlichen Daten der Verarbeitungs-Datengruppe IPP mittels Tastatur 13 und/oder Anzeigeeinheit 12.

e) Schreiben der eingegebenen Daten der Steuerungs-, Verarbeitungs- und Patienten-Datengruppe IPC, IPP bzw. IDP in den Speicher M des integrierten Schaltkreises 3 der Bildplatte 1 bzw. Kassette 2.

f) Schreiben von Daten der Zustands-Datengruppe IPS zum aktuellen Bearbeitungsstatus in den Speicher M: Bearbeitungsstatus wird auf "Röntgenbild aufgenommen" gesetzt.

Anschließend wird die Kassette 2 mit der darin befindlichen Bildplatte 1 zur Auslesevorrichtung 20 gebracht.

Dort wird die Bildplatte 1 mit dem Scanner 21 zeilenweise ausgelesen, wobei Bildsignale erzeugt werden, die den in der Bildplatte 1 gespeicherten Röntgeninformationen entsprechen. Die Bildsignale werden einer Vorverarbeitung unterzogen, bei der Bilddaten IMD erhalten werden, die an die Wiedergabevorrichtung 30 weitergegeben werden können. In der Auslesevorrichtung 20 werden dabei im Einzelnen folgende Schritte durchgeführt:

a) Lesen von Daten der Bildträger-, Zustands-, Steuerungs-, Verarbeitungs- und Patienten-Datengruppe IPI, IPS, IPC, IPP bzw. IDP aus dem Speicher M.

b) Lesen von Daten der Kalibrierungs-Datengruppe CAL, d.h. von Kalibrierdaten der Bildplatte 1, aus dem Speicher M. Oder:

Lesen von bereits bei einem früheren Auslesevorgang dieser Bildplatte 1 in einem Speicher (nicht dargestellt) der Auslesevorrichtung 20 gespeicherten Daten der Kalibrierungs-Datengruppe CAL.

c) Prüfen der gelesenen Daten der Zustands-Datengruppe IPS, ob der Bearbeitungsstatus der Bildplatte 1 auf "Röntgenbild aufgenommen" gesetzt ist. Andernfalls wird die weitere Bearbeitung abgebrochen.

d) Lesen von in einem Speicher der Auslesevorrichtung 20 gespeicherten Kalibrierdaten (CALS) des Scanners 21.

e) Zeilenweises Auslesen der in der Bildplatte 1 gespeicherten Röntgeninformationen mit dem Scanner 21 unter Heranziehung von Daten der Bildträger- und Steuerungs-Datengruppe IPI bzw. IPC und Erzeugen von entsprechenden Bildsignalen.

f) Vorverarbeitung der erzeugten Bildsignale anhand der Kalibrierdaten CAL der Bildplatte 1 und der Kalibrierdaten (CALS) des Scanners 21 zu Bilddaten IMD.

g) Löschen von etwaigen restlichen Röntgeninformationen in der Bildplatte 1 mit der Löscheinrichtung 23 unter Heranziehung von Daten der Bildträger- und Steuerungs-Datengruppe IPI bzw. IPC.

h) Speichern der Bilddaten IMD zusammen mit den Daten der Verarbeitungs- und Patienten-Datengruppe IPP bzw. IDP im Zentralspeicher 40.

i) Schreiben von Daten der Zustands-Datengruppe IPS zum aktuellen Bearbeitungsstatus der Bildplatte 1 in den Speicher M: "Bildplatte ausgelesen" bzw. "Bildplatte gelöscht".

Optional können in Schritt h) zusätzlich die Daten der Bildtärger- und/oder Steuerungs-Datengruppe IPI bzw. IPC zusammen mit den Bilddaten IMD im Zentralspeicher 40 gespeichert werden.

Die beim Auslesen der Bildplatte 1 erzeugten Bilddaten IMD können dann in der Wiedergabevorrichtung 30 auf einem Monitor 31 dargestellt und/oder auf einem Hardcopygerät 33 ausgegeben werden. Im Einzelnen werden hierbei folgende Schritte durchgeführt:

a) Lesen der Bilddaten IMD und der zugehörigen Daten der Verarbeitungs- und Patienten-Datengruppe IPP bzw. IDP aus dem Zentralspeicher 40.

b) Weiterverarbeitung der Bilddaten IMD auf der Grundlage der Daten der Verarbeitungs-Datengruppe IPP.

c) Wiedergabe - z.B. am Monitor 31 und/oder Hardcopygerät 33 - der weiterverarbeiteten Bilddaten IMD unter Heranziehung der Daten der Verarbeitungs- und/oder Patienten-Datengruppe IPP bzw. IDP.

In einer Abwandlung des in Fig. 3 dargestellten Datenflusses werden an der ID-Station 10 anstelle der Daten der Steuerungs-, Verarbeitungs- und Patienten-Datengruppe IPC, IPP bzw. IDP ein oder mehrere Referenzcodes IPC-Ref, IPP-Ref bzw. IDP-Ref in den Speicher M des integrierten Schaltkreises 3 der Bildplatte 1 bzw. Kassette 2 geschrieben. Die Referenzcodes IPC-Ref, IPP-Ref bzw. IDP-Ref werden zusammen mit den entsprechenden Daten der Steuerungs-, Verarbeitungs- und Patienten-Datengruppe IPC, IPP bzw. IDP im Zentralspeicher 40 gespeichert. Anhand der Referezcodes IPC-Ref, IPP-Ref bzw. IDP-Ref kann dann die Auslesevorrichtung 20 auf die im Zentralspeicher 40 gespeicherten Daten der entsprechenden Datengruppe IPC, IPP bzw. IDP zugreifen. Die bei dieser Abwandlung übertragenen Referenzcodes IPC-Ref, IPP-Ref bzw. IDP-Ref, ggf. mit den zugehörigen Daten der entsprechenden Datengruppe IPC, IPP bzw. IDP sind in der Fig. 3 in runde Klammern () gesetzt. Im übrigen gelten die obigen Ausführungen entsprechend.

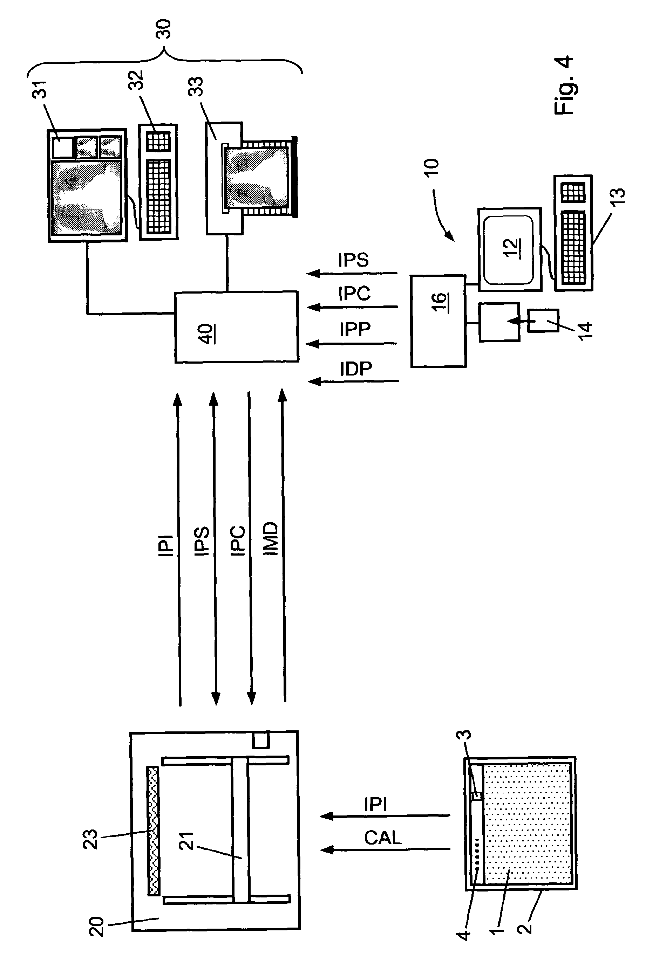

Fig. 4 zeigt eine schematische Darstellung einer zweiten Variante des Datenflusses zwischen den einzelnen Komponenten des in Fig. 1 dargestellten Systems. Bei dieser Variante des Datenflusses werden an der ID-Station 10 - wie oben näher beschrieben - Daten der Zustands-, Steuerungs-, Verarbeitungs- und Patienten-Datengruppe IPS, IPC, IPP bzw. IDP eingegeben, jedoch nicht in den Speicher M des auf der Bildplatte 1 befindlichen integrierten Schaltkreises 3 geschrieben, sondern an den Zentralspeicher 40 übertragen und dort gespeichert. Im Speicher M des integrierten Schaltkreises 3 sind bei dieser Variante lediglich Daten der Bildträger- und Kalibrierungs-Datengruppe IPI bzw. CAL gespeichert. Entsprechend werden in der Auslesevorrichtung 20 lediglich Daten der Bildträger- und Kalibrierungs-Datengruppe IPI bzw. CAL, welche bildplattenspezifische Daten bzw. die Kalibrierdaten der Bildplatte 1 umfassen, aus dem integrierten Schaltkreis 3 gelesen. Die Daten der Zustands-Datengruppe IPS zum Bearbeitungsstatus der Bildplatte 1 sowie die für das Auslesen der Röntgeninformation aus der Bildplatte 1 sowie das anschließende Löschen restlicher Bildinformation erforderlichen Daten der Steuerungs-Datengruppe IPC werden dagegen aus dem Zentralspeicher 40 gelesen. Im Übrigen gelten die Ausführungen zu Fig. 3 entsprechend.

Bei den in den Beispielen der Figuren 3 und 4 gezeigten Bildplatten 1 ist die Markierung 4, die einen Teil der im Speicher M des integrierten Schaltkreises 3 gespeicherten Daten, insbesondere Daten der Bildträger-Datengruppe IPI, repräsentiert, auf der Rückseite der Trägerschicht der Bildplatte 1 angebracht. Die Markierung 4 ist daher gestrichelt dargestellt.

Fig. 5 zeigt ein zweites Beispiel einer Struktur der im integrierten Schaltkreis 3 des Bildträgers gespeicherten Daten in der in Fig. 4 dargestellten Variante des Datenflusses. Wie bereits erläutert, sind bei dieser Variante lediglich Daten der Bildträger- und Kalibrierungs-Datengruppe IPI bzw. CAL im Speicher M gespeichert. Für die Struktur und den Inhalt der einzelnen Datengruppen IPI bzw. CAL gelten die obigen Ausführungen zu Fig. 2 entsprechend.

Bei dieser Variante kann der Speicherplatzbedarf im Speicher M des integrierten Schaltkreises gegenüber dem in den Figuren 2 und 3 gezeigten Beispiel reduziert werden.

Bei dem in den Figuren 2 und 3 gezeigten Beispiel dagegen wird mehr Speicherplatz benötigt, wobei jedoch die Auslesevorrichtung 20 die für das Auslesen bzw. Löschen erforderlichen Daten direkt aus dem Speicher M des integrierten Schaltkreises 3 auf der Bildplatte entnehmen kann und hierfür nicht auf den Zentralspeicher 40 zugreifen muss. Dadurch wird eine Unabhängigkeit der Auslesevorrichtung 20 von einem Zentralspeicher 40 erreicht.

## Patentansprüche

1. Verfahren zur Bearbeitung eines Bildträgers (1, 2) zur Aufzeichnung von Röntgenaufnahmen, wobei

   - der Bildträger (1, 2) einen elektronischen Speicher (M) zur Speicherung von Kalibrierdaten des Bildträgers (1, 2) umfasst, welche ein Maß für die Empfindlichkeit des Bildträgers (1, 2) für Röntgenstrahlung darstellen und bei einer Verarbeitung von Bildsignalen, welche bei einem Auslesen der Röntgeninformation aus dem Bildträger (1, 2) in einer Auslesevorrichtung (20) erhalten werden, herangezogen werden können,
   - die Kalibrierdaten nach einem ersten Auslesen der Kalibrierdaten aus dem elektronischen Speicher (M) des Bildträgers (1, 2), insbesondere durch eine ID-Station oder die Auslesevorrichtung (20), in einem Zentralspeicher (40) oder in einem Speicher der Auslesevorrichtung (20), gespeichert werden,
   - die Kalibrierdaten bei der Bearbeitung des Bildträgers (1, 2) in der Auslesevorrichtung (20) herangezogen werden, und
   - bei einer erneuten Bearbeitung desselben Bildträgers (1, 2) in der Auslesevorrichtung (20) die nach dem ersten Auslesen der Kalibrierdaten aus dem elektronischen Speicher (M) des Bildträgers (1, 2) im Zentralspeicher (40) bzw. im Speicher der Auslesevorrichtung (20), gespeicherten Kalibrierdaten dieses Bildträgers (1, 2) aus dem Zentralspeicher (40) bzw. dem Speicher der Auslesevorrichtung (20), gelesen werden.

2. Verfahren nach Anspruch 1, **dadurch gekennzeichnet, dass** das erste Auslesen der Kalibrierdaten des Bildträgers (1, 2) aus dem elektronischen Speicher (M) des Bildträgers (1, 2) in der Auslesevorrichtung (20) erfolgt.

3. Verfahren nach Anspruch 1 oder 2, **dadurch gekennzeichnet, dass** die Kalibrierdaten des Bildträgers (1, 2) ein Maß für eine über eine Speicherleuchtstoff-Schicht (5) des Bildträgers (1, 2) lokal veränderliche Empfindlichkeit des Bildträgers (1, 2) für Röntgenstrahlung darstellen.

4. Verfahren nach Anspruch 3, **dadurch gekennzeichnet, dass** die Kalibrierdaten des Bildträgers (1, 2) in einem zweidimensionalen Datenfeld im elektronischen Speicher (M) des Bildträgers (1, 2) gespeichert sind.

5. Verfahren nach Anspruch 3 oder 4 **dadurch gekennzeichnet, dass** die Kalibrierdaten des Bildträgers (1, 2) die lokal veränderliche Empfindlichkeit des Bildträgers (1, 2) in einzelnen Bereichen des Bildträgers (1, 2) wiedergeben, wobei die einzelnen Bereiche den einzelnen Bildpunkten entsprechen, die bei einem Auslesen der im Bildträger (1, 2) gespeicherten Röntgeninformation erhalten werden.

6. Verfahren nach Anspruch 3 oder 4, **dadurch gekennzeichnet, dass** die Kalibrierdaten des Bildträgers (1, 2) die lokal veränderliche Empfindlichkeit des Bildträgers (1, 2) in einzelnen Bereichen des Bildträgers wiedergeben, wobei die einzelnen Bereiche größer sind als die einzelnen Bildpunkte, die bei einem Auslesen der im Bildträger (1, 2) gespeicherten Röntgeninformation erhalten werden.

**7.** Verfahren nach einem der vorangehenden Ansprüche, **dadurch gekennzeichnet, dass** die Kalibrierdaten des Bildträgers (1, 2) in einer Kalibrierungs-Datengruppe (CAL) im elektronischen Speicher (M) des Bildträgers (1, 2) gespeichert sind.

**8.** Verfahren nach Anspruch 7, **dadurch gekennzeichnet, dass** die Kalibrierungs-Datengruppe (CAL) eine Versionsnummer (VN) umfasst, welche die Struktur, in welcher die Daten der Kalibrier-Datengruppe (CAL) vorliegen, kennzeichnet.

**9.** Verfahren nach Anspruch 7 oder 8, **dadurch gekennzeichnet, dass** die Kalibrierungs-Datengruppe (CAL) eine aus den Daten der Kalibrierungs-Datengruppe (CAL) abgeleitete Prüfsumme (CS) umfasst, anhand welcher überprüft werden kann, ob die Daten der Kalibrierungs-Datengruppe (CAL) fehlerfrei gespeichert und/oder gelesen worden sind.

**10.** Verfahren nach einem der vorangehenden Ansprüche, **dadurch gekennzeichnet, dass** die Übertragung von Daten zwischen dem elektronischen Speicher (M) des Bildträgers (1, 2) und der Auslesevorrichtung (20) kontaktlos erfolgt.

**11.** Verfahren nach einem der vorangehenden Ansprüche, **dadurch gekennzeichnet, dass** die Kalibrierdaten im elektronischen Speicher (M) des Bildträgers (1, 2) in komprimierter Form gespeichert sind.

**Claims**

**1.** A method for the processing of an image carrier (1, 2) for recording X-ray photos, wherein

- the image carrier (1, 2) comprises an electronic memory (M) for the storage of calibration data of the image carrier (1, 2) that represent a measure of the sensitivity of the image carrier (1, 2) to X-ray radiation and can be used when processing image signals that are obtained when reading out the X-ray information from the image carrier (1, 2) in a read-out device (20),
- the calibration data are stored in a central memory (40) or in a memory of the read-out device (20) after a first read-out of the calibration data from the electronic memory (M) of the image carrier (1, 2), in particular by means of an ID station or the read-out device (20),
- the calibration data are used when processing the image carrier (1, 2) in the read-out device (20), and
- when the same image carrier (1, 2) is processed again in the read-out device (20) the calibration data of this image carrier (1, 2) stored in the central memory (40) or in the memory of the read-out device (20) after the first read-out of the calibration data from the electronic memory (M) of the image carrier (1, 2) are read from the central memory (40) or from the memory of the read-out device (20).

**2.** Method according to claim 1, wherein the first read-out of the calibration data of the image carrier (1, 2) from the electronic memory (M) of the image carrier (1, 2) is performed in the read-out device (20).

**3.** Method according to claim 1 or 2, wherein the calibration data of the image carrier (1, 2) represent a measure of a sensitivity of the image carrier (1, 2) to X-ray radiation which is locally changing across a storage phosphor layer (5) of the image carrier (1, 2).

**4.** Method according to claim 3, wherein the calibration data of the image carrier (1, 2) are stored in a two-dimensional data field in the electronic memory (M) of the image carrier (1, 2).

**5.** Method according to claim 3 or 4, wherein the calibration data of the image carrier (1, 2) reproduce the locally changing sensitivity of the image carrier (1, 2) in individual areas of the image carrier (1, 2), the individual areas corresponding to the individual picture elements that are obtained during a read-out of the X-ray information stored in the image carrier (1, 2).

**6.** Method according to claim 3 or 4, wherein the calibration data of the image carrier (1, 2) reproduce the locally changing sensitivity of the image carrier (1, 2) in individual areas of the image carrier, the individual areas being larger than the individual picture elements that are obtained during a read-out of the X-ray information stored in the image carrier (1, 2).

**7.** Method according to any of the preceding claims, wherein the calibration data of the image carrier (1, 2) are stored in a calibration data group (CAL) in the electronic memory (M) of the image carrier (1, 2).

8. Method according to claim 7, wherein the calibration data group (CAL) comprises a version number (VN) that characterises the structure in which the data of the calibration data group (CAL) are present.

9. Method according to claim 7 or 8, wherein the calibration data group (CAL) comprises a check sum (CS) derived from the data of the calibration data group (CAL), on the basis of which it can be checked whether the data of the calibration data group (CAL) have been stored and/or read in an error-free way.

10. Method according to any of the preceding claims, wherein the data transfer between the electronic memory (M) of the image carrier (1, 2) and the read-out device (20) is performed in a contactless way.

11. Method according to any of the preceding claims, wherein the calibration data are stored in compressed form in the electronic memory (M) of the image carrier (1, 2).

**Revendications**

1. Procédé de traitement d'un support d'image (1, 2) pour l'enregistrement de radiographies,

- dans lequel le support d'image (1, 2) comprend une mémoire électronique (M) pour la mémorisation de données de calibrage du support d'image (1, 2) qui représentent une mesure de la sensibilité du support d'image (1, 2) pour des rayons X et qui peuvent être exploitées lors d'un traitement de signaux vidéo qui sont obtenus lors d'une lecture de l'information radiographique sur le support d'image (1, 2) dans un dispositif de lecture (20),
- dans lequel les données de calibrage après une première lecture des données de calibrage dans la mémoire électronique (M) du support d'image (1, 2) sont mémorisées, notamment au moyen d'un poste d'identification ID ou du dispositif de lecture (20), dans une mémoire centrale (40) ou dans une mémoire du dispositif de lecture (20),
- dans lequel les données de calibrage sont exploitées lors du traitement du support d'image (1, 2) dans le dispositif de lecture (20), et
- dans lequel, lors d'un nouveau traitement du même support d'image (1, 2) dans le dispositif de lecture (20), les données de calibrage de ce support de données (1, 2) qui ont été mémorisées dans la mémoire centrale (40) ou dans la mémoire du dispositif de lecture (20) après la première lecture des données de calibrage dans la mémoire électronique (M) du support d'image (1, 2) sont lues dans la mémoire centrale (40) ou dans la mémoire du dispositif de lecture (20).

2. Procédé selon la revendication 1, **caractérisé par le fait que** la première lecture des données de calibrage du support d'image (1, 2) dans la mémoire électronique (M) du support d'image (1, 2) s'effectue dans le dispositif de lecture (20).

3. Procédé selon la revendication 1 ou 2, **caractérisé par le fait que** les données de calibrage du support d'image (1, 2) représentent une mesure de la sensibilité du support d'image (1, 2) pour des rayons X, laquelle sensibilité est localement variable sur une couche luminescente de mémorisation (5) du support d'image (1, 2).

4. Procédé selon la revendication 3, **caractérisé par le fait que** les données de calibrage du support d'image (1, 2) sont mémorisées dans un champ de données bidimensionnel dans la mémoire électronique (M) du support d'image (1, 2).

5. Procédé selon la revendication 3 ou 4, **caractérisé par le fait que** les données de calibrage du support d'image (1, 2) reproduisent la sensibilité localement variable du support d'image (1, 2) dans différentes zones du support d'image (1, 2), les zones individuelles correspondant à des points d'image individuels qui sont obtenus lors d'une lecture de l'information radiographique mémorisée dans le support d'image (1, 2).

6. Procédé selon la revendication 3 ou 4, **caractérisé par le fait que** les données de calibrage du support d'image (1, 2) reproduisent la sensibilité localement variable du support d'image (1, 2) dans différentes zones du support d'image, les zones individuelles étant plus grandes que les points d'image individuels qui sont obtenus lors d'une lecture de l'information radiographique mémorisée dans le support d'image (1, 2).

**7.** Procédé selon l'une des revendications précédentes, **caractérisé par le fait que** les données de calibrage du support d'image (1, 2) sont mémorisées dans un groupe de données de calibrage (CAL) dans la mémoire électronique (M) du support d'image (1, 2).

**8.** Procédé selon la revendication 7, **caractérisé par le fait que** le groupe de données de calibrage (CAL) comprend un numéro de version (VN) qui caractérise la structure dans laquelle se trouvent les données du groupe de données de calibrage (CAL).

**9.** Procédé selon la revendication 7 ou 8, **caractérisé par le fait que** le groupe de données de calibrage (CAL) comprend une somme de contrôle (CS) qui est déduite des données du groupe de données de calibrage (CAL) et à l'aide de laquelle on peut vérifier si les données du groupe de données de calibrage (CAL) ont été mémorisées et/ou lues sans erreur.

**10.** Procédé selon l'une des revendications précédentes, **caractérisé par le fait que** la transmission de données entre la mémoire électronique (M) du support d'image (1, 2) et le dispositif de lecture (20) s'effectue sans contact.

**11.** Procédé selon l'une des revendications précédentes, **caractérisé par le fait que** les données de calibrage sont mémorisées sous forme compressée dans la mémoire électronique (M) du support d'image (1, 2).

Fig. 1

M

IPI    IPS    IPC    IPP

| VN | VN | VN | VN |
|----|----|----|----|
| ... | ... | ... | ... |
| .... | .... | .... | .... |
| .... | .... | .... | .... |
| CS | CS | CS | CS |

CAL    IDP

| VN | | VN |
|----|----|----|
| ... | | ... |
| .... | | .... |
| .... | | .... |
| CS | | CS |

Fig. 2

M

IPI

| VN |
|----|
| ... |
| .... |
| .... |
| CS |

CAL

| VN |
|----|
| ... |
| .... |
| .... |
| CS |

Fig. 5

30

31
32
33

10

(IPC + IPC-Ref)
(IPP + IPP-Ref)
(IDP + IDP-Ref)

16

12

13

14

40

IPI
IPS
IPC (IPC-Ref)
IPP (IPP-Ref)
IDP (IDP-Ref)
IMD

IPS
IPC (IPC-Ref)
IPP (IPP-Ref)
IDP (IDP-Ref)

IPI
IPS
IPC (IPC-Ref)
IPP (IPP-Ref)
IDP (IDP-Ref)
CAL

23
21
20

3
4
1
2

Fig. 3

EP 1 544 674 B1

20  23

21

IPI

IPS

IPC

IMD

40

31

32

30

33

CAL  IPI

IDP  IPP  IPC  IPS

16

10

4  3

1

2

14

12

13

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1391780 A1
- US 4320296 A
- EP 1031854 A1
- EP 1251683 A1
- EP 0727696 A1
- WO 0242794 A2